# EUROPEAN PATENT APPLICATION

(11) **EP 2 500 011 A1**
(43) Date of publication of application: **19.09.2012**
(21) Application number: 10828379.7
(22) Date of filing: 08.11.2010
(51) Int. Cl.: A61K 8/31, A61K 8/02, A61Q 1/04, A61Q 1/06

(54) **STICK-TYPE COSMETIC PREPARATION AND METHOD FOR PRODUCING SAME**

(30) Priority: 09.11.2009 JP 2009255783
(71) Applicant: SHISEIDO COMPANY, LTD., Tokyo 104-8010 (JP)
(72) Inventor: HOSOKAWA, Kinya, Yokohama-shi Kanagawa 224-8558 (JP); ABE, Masami, Kamakura-shi Kanagawa 247-0051 (JP); YAMASHITA, Hisayoshi, Tokyo 104-8010 (JP); ASADA, Osamu, Kakegawa-shi Shizuoka 436-0047 (JP)
(74) Representative: Ulmann, Catherine Claire
(86) International application number: PCT/JP2010/069848
(87) International publication number: WO 2011/055821

(57) **Abstract**

A stick-type cosmetic includes a solid wax whose content is more than or equal to 5% by weight and less than or equal to 8% by weight, wherein hardness of the stick-type cosmetic is more than or equal to 0.1 and less than or equal to 0.4, and gloss of the stick-type cosmetic is more than or equal to 30.

## Description

### TECHNICAL FIELD

The present invention relates to a stick-type cosmetic and a method of manufacturing a stick-type cosmetic.

### BACKGROUND ART

Conventionally, a lipstick in which 3% of polyethylene (MW=700), 1% of candelilla wax, 40% of glyceryl isostearate, 46% of liquid paraffin, 4% of titanium oxide, 3% of titanium oxide-mica, 3% of organic powders and dosage of flavor are blended, is known (Patent Document 1).

The lipstick is generally manufactured by heating and melting a composition for lipstick, filling a mold (die) or ogive with the composition for lipstick, and cooling. However, when the content of the solid wax in the composition for lipstick is as small as described in Patent Document 1, there is a problem that formability, shape-retaining ability and glossiness of the lipstick are lowered.

On the other hand, when the content of the solid wax in the composition for lipstick is large, there is a problem that sensation of the lipstick is lowered.

### [Patent Document]

[Patent Document 1] Japanese Laid-open Patent Publication No. S58-15904

### SUMMARY OF THE INVENTION

The present invention is made in light of the above problems, and provides a stick-type cosmetic and a method of manufacturing a stick-type cosmetic good at sensation, formability, shape-retaining ability and glossiness.

According to an embodiment, there is provided a stick-type cosmetic including a solid wax whose content in the stick-type cosmetic is more than or equal to 5% by weight and less than or equal to 8% by weight, and wherein hardness of the stick-type cosmetic is more than or equal to 0.1 and less than or equal to 0.4, and gloss of the stick-type cosmetic is more than or equal to 30.

According to another embodiment, there is provided a method of manufacturing a stick-type cosmetic, including heating and melting a composition for the stick-type cosmetic in which the content of a solid wax is more than or equal to 5% by weight and less than or equal to 8% by weight; supplying the heated and melted composition for stick-type cosmetic in an elastic mold; and cooling the composition for the stick-type cosmetic in the elastic mold, wherein a product of the thickness and the elastic coefficient of the elastic mold is more than or equal to 0.15 N/mm and less than or equal to 0.5 N/mm.

According to the embodiment, a stick-type cosmetic and a method of manufacturing a stick-type cosmetic good at sensation, formability, shape-retaining ability and glossiness are provided.

### BRIEF DESCRIPTION OF DRAWINGS

Other objects, features and advantages of the present invention will become more apparent from the following detailed description when read in conjunction with the accompanying drawings.

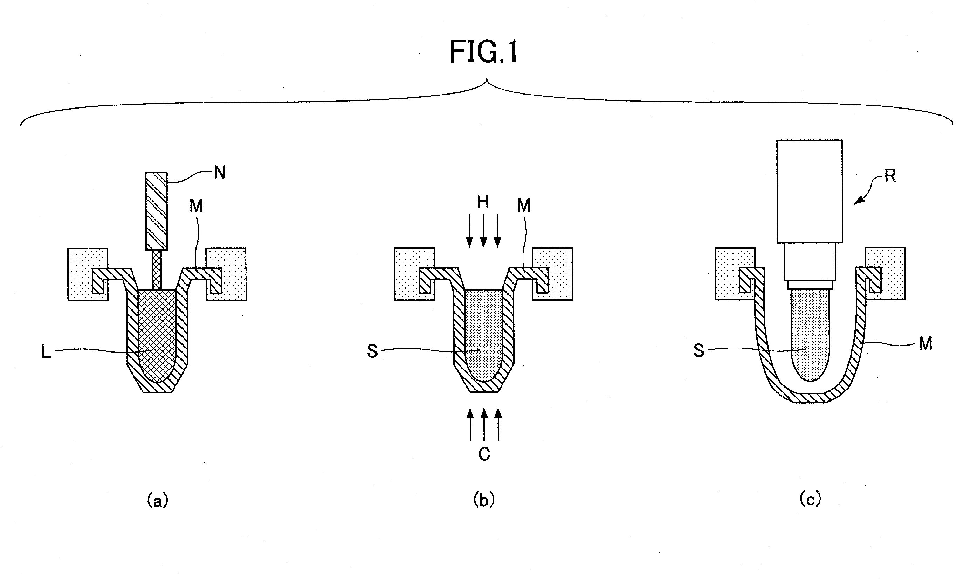

Fig. 1 is a view for explaining a method of manufacturing a stick-type cosmetic of an embodiment.

### DETAILED DESCRIPTION OF THE PREFERRED EMBODIMENTS

An example of the embodiment is explained with reference to Fig. 1.

According to a stick-type cosmetic of the embodiment, the content of a solid wax is 5 to 8% by weight, preferably may be 5.5 to 8% by weight, and more preferably may be 5.5 to 7% by weight. When the content of the solid wax is less than 5% by weight, formability and shape-retaining ability of the stick-type cosmetic are lowered, while when the content of the solid wax exceeds 8% by weight, sensation of the stick-type cosmetic is lowered.

Further, for the stick-type cosmetic of the embodiment, hardness is between 0.1 to 0.4, preferably may be between 0.15 to 0.4, and more preferably may be 0.15 to 0.35. When the hardness is less than 0.1, formability and shape-retaining ability of the stick-type cosmetic are lowered, while when the hardness exceeds 0.4, sensation of the stick-type cosmetic is lowered.

Further, for the stick-type cosmetic of the embodiment, gloss is more than or equal to 30, preferably may be more than or equal to 35, and more preferably may be more than or equal to 40. When the gloss is less than 30, an attractive appearance as a stick-type cosmetic is lowered.

Here, the hardness may be measured by using a rheometer. Further, the gloss may be measured by using a gloss meter.

Material for the solid wax is not limited provided that it is in a solid state at 15 °C, but for the solid wax, polyethylene wax, Fischer-Tropsch Wax, paraffin wax, microcrystalline wax, candelilla wax, carnauba wax, rice wax or the like may be used, and two or more of these may be combined to be used. Among these, polyethylene wax may be preferably used as an amount of adding can be reduced and more preferably, polyethylene wax with a weight average molecular weight of 500 to 700 may be used.

For the commercially available polyethylene wax, PERFORMALENE 500/PL/655 (manufactured by New Phase Technologies), A-C1702 (manufactured by Honeywell), Epolene-N34·35 (manufactured by Eastman Chemical Company) or the like may be used.

The stick-type cosmetic of the embodiment may preferably further include a liquid oil ingredient.

Material for the liquid oil ingredient is not limited provided that it is in a liquid state at 15 °C, but for the liquid oil ingredient, neopentyl glycol dicaprate, triethylhexanoin, pentaerythrityl tetraethylhexanoate, caprylic/capric triglyceride, diethylhexyl sebacate, octyldodecanol, glyceryl diisostearate, diglyceryl triisostearate, diisostearyl malate, trimethylolpropane triethylhexanoate, oxystearic acid oxystearyl, pentaerythrityl tetraethylhexanoate/benzoate, castor oil, dimethyl polysiloxane, decamethyl pentasiloxane, macadamia nut oil, jojoba oil, squalane, liquid lanolin, glyceryl tri (hydrogenated rosin·isostearate), methylphenyl poly siloxane, glyceryl triisostearate, polybutene, polyisobutene or the like may be used, and two or more of these may be combined to be used. Among these, liquid lanolin, glyceryl tri (hydrogenated rosin· isostearate), methylphenyl poly siloxane, glyceryl triisostearate or polybutene may be preferably used.

The content of the liquid oil ingredient in the stick-type cosmetic of the embodiment may be, generally, 30 to 95% by weight, and preferably may be 50 to 95% by weight. When the content of the liquid oil ingredient is less than 30% by weight, sensation of the stick-type cosmetic may be lowered, while when the content of the liquid oil ingredient exceeds 95% by weight, formability and shape-retaining ability of the stick-type cosmetic may be lowered.

In this embodiment, liquid oil ingredient and semisolid oil ingredient may be combined to be used. The semisolid oil ingredient is not limited as long as it is in a semisolid state at 15 °C, but Cosmol 168M (manufactured by The Nisshin Oillio Group, Ltd.), which is dipentaerythrityl hexahydroxystearate, or the like may be used, and two or more materials may be combined to be used.

The stick-type cosmetic of the embodiment may further include, in accordance with necessity, humectants, preservatives, antioxidants, ultraviolet absorbents, high molecular compounds, surfactants, dye, pigment, powders, agents, alcohol, solvent, flavor or the like.

Although not limited to, but the stick-type cosmetic of the embodiment may be applicable for a cosmetic for lips such as a lipstick, a lip gloss, a lip cream or the like; a make-up cosmetic such as a foundation, an eye shadow or the like; a hair cosmetic such as a hair stick, a pomade or the like may be used. Among these, a cosmetic for lips that applies sensation may be preferably used, and a lipstick including a powder coloring material may be more preferably used.

Next, a method of manufacturing the stick-type cosmetic of the embodiment is explained. First, melted liquid L which is obtained by heating and melting while mixing a composition for stick-type cosmetic in which the content of a solid wax is more than or equal to 5% by weight and less than or equal to 8% by weight, is supplied into an elastic mold M from a filling nozzle N (see Fig. 1(a)). At this time, the elastic mold M is preheated by spraying warm air. Then, a stick-type cosmetic S is shaped by spraying cool air C from a lower side of the elastic mold M to cool the melted liquid L while re-heating the melted liquid L by spraying hot air H from an upper side of the elastic mold M, (see Fig. 1(b)). Then, after the stick-type cosmetic S is attached to a container R, the elastic mold M is opened and stick-type cosmetic S is taken out (see Fig. 1(c)).

At this time, a product of a thickness and elastic coefficient of the elastic mold M may preferably be 0.15 to 0.5 N/mm. When the product of the thickness and the elastic coefficient of the elastic mold M is less than 0.15 N/mm, it is difficult to form the stick-type cosmetic S because the elastic mold M is soft, while when the product of the thickness and the elastic coefficient of the elastic mold M exceeds 0.5 N/mm, sensation, formability, shape-retaining ability and glossiness of the stick-type cosmetic S are lowered.

When the melted liquid L filling the elastic mold M is cooled, the elastic mold M follows a shrinkage of the melted liquid L, and the melted liquid L is uniquely caked to prevent a generation of a distortion of the stick-type cosmetic S. As a result, it can be estimated that the stick-type cosmetic S which is good at sensation, formability, shape-retaining ability and glossiness is obtained.

For an elastic body that composes the elastic mold M, not specifically limited, but a rubber such as a silicone rubber or the like may be used.

Next, with some examples, the embodiment will be specifically explained. The present invention is not limited by the following examples. Here, "part" means "part by weight".

### [Example 1]

A composition for lipstick composed of 5% by weight of PERFORMALENE 655 (manufactured by New Phase Technologies) which is polyethylene wax, 30% by weight of pentaerythrityl tetraethylhexanoate/benzoate, 10% by weight of diisostearyl malate, 10% by weight of castor oil, 5% by weight of 3-(4-Methoxyphenyl)propionic acid 2-ethylhexyl ester, 36.5% by weight of pentaerythrityl tetraethylhexanoate, 0.1% by weight of Red 202, 1.5% by weight of colcothar, 0.5% by weight of yellow iron oxide, 1.2% by weight of titanium oxide, 0.1% by weight of tocopherol and 0.1% by weight of flavor was agitated and mixed for 10 minutes at 100 °C, supplied into the elastic mold M after defoaming, and cooled to 5 °C to obtain a lipstick (see Fig. 1). The hardness of the obtained lipstick was 0.13, and the gloss of the obtained lipstick was 48.

At this time, a silicone rubber whose thickness was 1.6 mm and whose elastic coefficient was 0.184 N/mm² was used as the elastic mold M, and the elastic mold M was pre-heated for two minutes at 120 °C. Further, the melted liquid L was cooled by being left standing for four minutes at 0 °C after spraying cool air C at -12 °C onto the elastic mold M for four minutes. Further, when spraying the cool air C onto the elastic mold M, air at a room temperature was sprayed for two minutes after hot air H at 120 °C was sprayed for 40 seconds for re-heating.

### [Example 2]

A lipstick was obtained by the same method as the example 1 except that the contents of polyethylene wax and pentaerythrityl tetraethylhexanoate were altered to 5.5% by weight and 36% by weight, respectively. The hardness of the obtained lipstick was 0.17, and the gloss of the obtained lipstick was 47.

### [Example 3]

A lipstick was obtained by the same method as the example 1 except that the contents of polyethylene wax and pentaerythrityl tetraethylhexanoate were altered to 8% by weight and 33.5% by weight, respectively. The hardness of the obtained lipstick was 0.36, and the gloss of the obtained lipstick was 45.

### [Example 4]

A lipstick was obtained by the same method as the example 3 except that Fischer-Tropsch Wax was added instead of the polyethylene wax. The hardness of the obtained lipstick was 0.17, and the gloss of the obtained lipstick was 38.

### [Relative example 1]

A lipstick was obtained by the same method as the example 1 except that the contents of polyethylene wax and pentaerythrityl tetraethylhexanoate were altered to 4.5% by weight and 37% by weight, respectively. The hardness of the obtained lipstick was 0.09, and the gloss of the obtained lipstick was 39.

### [Relative example 2]

A lipstick was obtained by the same method as the example 1 except that the contents of polyethylene wax and pentaerythrityl tetraethylhexanoate were altered to 9% by weight and 32.5% by weight, respectively. The hardness of the obtained lipstick was 0.46, and the gloss of the obtained lipstick was 40.

### [Relative example 3]

A lipstick was obtained by the same method as the example 2 except that a resin mold was used instead of the elastic mold M. The hardness of the obtained lipstick was 0.20, and the gloss of the obtained lipstick was 25.

### [Relative example 4]

A lipstick was obtained by the same method as the example 3 except that a resin mold was used instead of the elastic mold M. The hardness of the obtained lipstick was 0.29, and the gloss of the obtained lipstick was 28.

### [Measurement of hardness]

Hardness was measured by using a rheometer RTC-2002 D-D (manufactured by RHEOTECH) on samples which had been left standing for 1 hour at 30 °C, under a condition where a diameter of a pressure sensing shaft was 1 mm, a penetration speed was 2 cm / minute, and a penetration degree was 3 mm.

### [Measurement of gloss]

Gloss was measured by using a gloss meter VG-2000 (manufactured by NIPPON DENSHOKU INDUSTRIES CO., LTD.) under a condition where an incident angle was 60°.

Then, formability, shape-retaining ability, spreadability when applying, luster and glossiness of the lipsticks of the examples 1 to 4 and the relative examples 1 to 4 were evaluated. The evaluated results are shown in Table 1.

| | CONTENT OF SOLID WAX [% BY WEIGHT] | HARDNESS | GLOSS | FORMABILITY | SHAPE-RETAINING ABILITY | SPREADABILITY WHEN APPLYING | LUSTER | GLOSSINESS |
|---|---|---|---|---|---|---|---|---|
| EXAMPLE 1 | 5.0 | 0.13 | 48 | O | Δ | ⊚ | ⊚ | ⊚ |
| EXAMPLE 2 | 5.5 | 0.17 | 47 | ⊚ | O | ⊚ | ⊚ | ⊚ |
| EXAMPLE 3 | 8.0 | 0.36 | 45 | ⊚ | O | O | O | ⊚ |
| EXAMPLE 4 | 8.0 | 0.17 | 38 | O | O | O | O | O |
| RELATIVE EXAMPLE 1 | 4.5 | 0.09 | 39 | Δ | × | ⊚ | ⊚ | O |
| RELATIVE EXAMPLE 2 | 9.0 | 0.46 | 40 | ⊚ | O | Δ | Δ | O |
| RELATIVE EXAMPLE 3 | 5.5 | 0.20 | 25 | × | × | ⊚ | ⊚ | × |
| RELATIVE EXAMPLE 4 | 8.0 | 0.29 | 28 | O | Δ | O | O | Δ |

As shown in Table 1, it can be understood that the lipsticks of the examples 1 to 4 are superior in sensation, formability, shape-retaining ability and glossiness.

### [Formability]

Percent defective (peeling, unevenness) of 100 lipsticks for each of the examples were obtained. Here, for the example for which the percent defective is less than 5% is shown double circles, for the example for which the percent defective is more than or equal to 5% and less than 10% is shown a single circle, for the example for which the percent defective is more than or equal to 10% and less than 30% is shown a triangle, and for the example for which the percent defective is more than or equal to 30% is shown × to be evaluated.

### [Shape-retaining ability]

Professional panelists (20 members) used the lipsticks, which were kept at 25 °C, and evaluated whether each of the lipsticks was broken. Here, for the lipstick for which the number of members among 20 members who reported that the lipstick was broken is zero is shown a circle, for the lipstick for which the number of members among 20 members who reported that the lipstick was broken is 1 to 4 is shown a triangle, and for the lipstick for which the number of members among 20 members who reported that the lipstick was broken is 5 or more is shown × to be evaluated.

### [Spreadability when applying]

Professional panelists (10 members) used the lipsticks, and conducted a sensory evaluation on a 5-point scale where 5 points for spreadability when applying is light, 4 points for a bit light, 3 points for ordinary, 2 points for a bit heavy, and 1 point for heavy. Here, for the lipstick whose average score of the sensory evaluation is more than or equal to 4.0 and less than or equal to 5.0 is shown double circles, for more than or equal to 3.0 and less than 4.0 is shown a circle, for more than or equal to 2.0 and less than 3.0 is shown a triangle, and for more than or equal to 1.0 and less than 2.0 is shown × to be evaluated.

### [Luster]

Professional panelists (10 members) used the lipsticks and conducted a sensory evaluation on a 5-point scale awarding 5 points for with luster, 4 points for with a bit of luster, 3 points for ordinary in luster, 2 points for less luster, and 1 point for no luster. Here, for the lipstick whose average score of the sensory evaluation is more than or equal to 4.0 and less than or equal to 5.0 is shown double circles, for more than or equal to 3.0 and less than 4.0 is shown a circle, for more than or equal to 2.0 and less than 3.0 is shown a triangle, and for more than or equal to 1.0 and less than 2.0 is shown × to be evaluated.

### [Glossiness]

Professional panelists (10 members) used the lipsticks and conducted a sensory evaluation on a 5-point scale awarding 5 points for with glossiness, 4 points for with a bit of glossiness, 3 points for ordinary in glossiness, 2 points for less glossiness, and 1 point for no glossiness. Here, for the lipstick whose average score of the sensory evaluation is more than or equal to 4.0 and less than or equal to 5.0 is shown double circles, for more than or equal to 3.0 and less than 4.0 is shown a circle, for more than or equal to 2.0 and less than 3.0 is shown a triangle, and for more than or equal to 1.0 and less than 2.0 is shown × to be evaluated.

### [Example 5]

A lipstick was obtained by the same method as the example 2 except that a silicone rubber whose thickness was 2.3 mm and whose elastic coefficient was 0.184 N/mm² was used as the elastic mold M. The hardness of the obtained lipstick was 0.17, and the gloss of the obtained lipstick was 40.

### [Example 6]

A lipstick was obtained by the same method as the example 2 except that a silicone rubber whose thickness was 1.0 mm and whose elastic coefficient was 0.184 N/mm² was used as the elastic mold M. The hardness of the obtained lipstick was 0.17, and the gloss of the obtained lipstick was 51.

### [Relative example 5]

A lipstick was obtained by the same method as the example 2 except that a silicone rubber whose thickness was 3.0 mm and whose elastic coefficient was 0.184 N/mm² was used as the elastic mold M. The hardness of the obtained lipstick was 0.17, and the gloss of the obtained lipstick was 28.

### [Relative example 6]

A lipstick was obtained by the same method as the example 2 except that a silicone rubber whose thickness was 1.6 mm and whose elastic coefficient was 0.803 N/mm² was used as the elastic mold M. The hardness of the obtained lipstick was 0.17, and the gloss of the obtained lipstick was 23.

### [Relative example 7]

A lipstick was obtained by the same method as the example 2 except that a silicone rubber whose thickness was 1.6 mm and whose elastic coefficient was 3.623 N/mm² was used as the elastic mold M. The hardness of the obtained lipstick was 0.17, and the gloss of the obtained lipstick was 15.

Then, glossiness of the lipsticks of the examples 5, 6 and the relative examples 5 to 7 were evaluated. The evaluated results are shown in Table 2.

| | ELASTIC MOLD | | | CONTENT OF SOLID WAX [% BY WEIGHT] | HARDNESS | GLOSS | GLOSSINESS |
|---|---|---|---|---|---|---|---|
| | THICKNESS [mm] | ELASTIC COEFFICIENT [N/mm²] | THICKNESS-(ELASTIC COEFFICIENT) [N/mm] | | | | |
| EXAMPLE 2 | 1.6 | 0.184 | 0.29 | 5.5 | 0.17 | 47 | ⊚ |
| EXAMPLE 5 | 2.3 | 0.184 | 0.42 | 5.5 | 0.17 | 40 | O |
| EXAMPLE 6 | 1.0 | 0.184 | 0.18 | 5.5 | 0.17 | 51 | ⊚ |
| RELATIVE EXAMPLE 5 | 3.0 | 0.184 | 0.55 | 5.5 | 0.17 | 28 | Δ |
| RELATIVE EXAMPLE 6 | 1.6 | 0.803 | 1.28 | 5.5 | 0.17 | 23 | × |
| RELATIVE EXAMPLE 7 | 1.6 | 3.623 | 5.80 | 5.5 | 0.17 | 15 | × |

As shown in table 2, it can be understood that the lipsticks of the examples 2, 5 and 6 are superior in glossiness.

### [Example 7]

A lipstick was obtained by the same method as the example 1 except that a composition for lipstick composed of 5% by weight of PERFORMALENE PL (manufactured by New Phase Technologies) which is polyethylene wax, 1% by weight of carnauba wax, 20% by weight of pentaerythrityl tetraethylhexanoate/benzoate, 15% by weight of castor oil, 2% by weight of sunflower oil, 10% by weight of dimethyl polysiloxane, 3% by weight of sheet hydroxy-apatite, 10% by weight of mica, 1% by weight of colcothar, 2% by weight of titanium oxide, 0.1% by weight of Red 202, 0.02% by weight of Red 218, 0.1% by weight of vitamin A palmitate, 0.1% by weight of tocopherol, 0.1% by weight flavor and a balance of neopentyl glycol dicaprate was agitated and mixed for 10 minutes at 95 °C. The hardness of the obtained lipstick was 0.23, the gloss of the obtained lipstick was 45 and the lipstick was superior in sensation, formability, shape-retaining ability and glossiness.

### [Example 8]

A lipstick was obtained by the same method as the example 1 except that a composition for lipstick composed of 6% by weight of PERFORMALENE 655 (manufactured by New Phase Technologies) which is polyethylene wax, 10% by weight of polyisobutene, 2.0% by weight of liquid lanolin, 10% by weight of phytosteryl/octyldodecyl lauroyl glutamate, 20% by weight of diisostearyl malate, 10% by weight of glyceryl diisostearate, 10% by weight of isostearyl hydroxystearate, 5% by weight of octyl methoxycinnamate, 1% by weight of barium sulfate, 1% by weight of colcothar coated titanated mica, 3% by weight of titanium oxide, 0.5% by weight of Red 202, 0.1% by weight of Red 201, 0.1% by weight of tocopherol, 0.1% by weight of flavor and a balance of triethylhexanoin was agitated and mixed for 10 minutes at 95 °C. The hardness of the obtained lipstick was 0.20, the gloss of the obtained lipstick was 47 and the lipstick was superior in sensation, formability, shape-retaining ability and glossiness.

### [Example 9]

A lipstick was obtained by the same method as the example 1 except that a composition for lipstick composed of 6% by weight of PERFORMALENE 655 (manufactured by New Phase Technologies) which is polyethylene wax, 2% by weight of candelilla wax, 10% by weight of squalane, 20% by weight of castor oil, 10% by weight of glyceryl triisostearate, 5% by weight of trimethyl pentaphenyl trisiloxane, 0.1% by weight of tocopherol acetate, 0.5% by weight of Red 201, 0.3% by weight of Red 202, 1.5% by weight of Yellow 4, 1% by weight of titanium oxide, 1% by weight of carmine coated titanated mica, 0.1% by weight of tocopherol, 0.1% by weight of flavor and a balance of neopentyl glycol dicaprate was agitated and mixed for 10 minutes at 95 °C. The hardness of the obtained lipstick was 0.28, the gloss of the obtained lipstick was 39 and the lipstick was superior in sensation, formability, shape-retaining ability and glossiness.

### [Example 10]

A lipstick was obtained by the same method as the example 1 except that a composition for lipstick composed of 5.5% by weight of PERFORMALENE 655 (manufactured by New Phase Technologies) which is polyethylene wax, 1% by weight of synthetic wax, 10% by weight of synthetic isoparaffin, 30% by weight of triethylhexanoin, 10% by weight of diisostearyl malate, 10% by weight of methylphenyl poly siloxane, 5% by weight of olive oil, 0.1% by weight of lecithin, 1% by weight of spherical cellulose powders, 2.5% by weight of titanium oxide coated glass powders, 0.8% by weight of yellow iron oxide, 1.1% by weight of Red 202, 2% by weight of titanium oxide, 1% by weight of titanated mica, 0.1% by weight of tocopherol and a balance of glyceryl diisostearate was agitated and mixed for 10 minutes at 90 °C. The hardness of the obtained lipstick was 0.25, the gloss of the obtained lipstick was 41 and the lipstick was superior in sensation, formability, shape-retaining ability and glossiness.

### [Example 11]

A lipstick was obtained by the same method as the example 1 except that a composition for lipstick composed of 6% by weight of PERFORMALENE 655 (manufactured by New Phase Technologies) which is polyethylene wax, 5% by weight of polybutene, 15% by weight of glyceryl triisostearate, 15% by weight of pentaerythrityl tetraethylhexanoate, 10% by weight of squalane, 0.5% by weight of calcium hydrogen phosphate, 2.1% by weight of colcothar, 0.1% by weight of black iron oxide, 2.3% by weight of titanium oxide, 0.2% by weight of Blue 1, 0.1% by weight of tocopherol and a balance of liquid paraffin was agitated and mixed for 10 minutes at 90 °C. The hardness of the obtained lipstick was 0.21, the gloss of the obtained lipstick was 44 and the lipstick was superior in sensation, formability, shape-retaining ability and glossiness.

### [Example 12]

After 3 parts of cholesteryl macadamiate, 0.5 parts of dimethiconepolyol and 10 parts of methylphenyl poly siloxane were agitated and mixed while being heated at 70 °C, a mixture obtained by dissolving 0.01 parts of edetate trisodium and 0.2 parts of glycerin in 1.5 parts of purified water was added to be dispersed to obtain an emulsifying base.

Then, the emulsifying base was added to a mixture solution obtained by agitating and mixing 7 parts of PERFORMALENE 655 (manufactured by New Phase Technologies) which is polyethylene wax, 35 parts of triethylhexanoin, 10 parts of hydrogenated-polybutene, 5 parts of ethylhexyl methoxycinnamate, 0.1 parts of dibutylhydroxytoluene, 0.1 parts of tocopherol acetate, 2 parts of colcothar, 4 parts of titanium dioxide, 0.1 parts of tocopherol, 0.1 parts of flavor and 21.39 parts of trimethylolpropane triethylhexanoate while being heated at 95 °C, agitated and mixed, supplied into an elastic mold after defoaming, and cooled to 5 °C to obtain a lipstick. The hardness of the obtained lipstick was 0.19, the gloss of the obtained lipstick was 47 and the lipstick was superior in sensation, formability, shape-retaining ability and glossiness.

### [Example 13]

A lip cream was obtained by the same method as the example 1 except that a composition for lip cream composed of 8% by weight of PERFORMALENE 655 (manufactured by New Phase Technologies) which is polyethylene wax, 10% by weight of vaseline, 10% by weight of dipentaerythrityl hexahydroxystearate, 15% by weight of diglyceryl triisostearate, 10% by weight of diisostearyl malate, 0.2% by weight of tocopherol acetate, 0.05% by weight of L-menthol, 0.1% by weight of tocopherol and a balance of liquid paraffin was agitated and mixed for 10 minutes at 85 °C. The hardness of the obtained lip cream was 0.29, the gloss of the obtained lip cream was 42 and the lip cream was superior in sensation, formability, shape-retaining ability and glossiness.

The present application is based on Japanese Priority Application No. 2009-255783 filed on November 9, 2009, the entire contents of which are hereby incorporated herein by reference.

### DESCRIPTION OF MARKS AND NUMERALS

- L: melted liquid
- N: filling nozzle
- M: elastic mold
- H: hot air
- C: cool air
- S: stick-type cosmetic
- R: container

## Claims

1. A stick-type cosmetic comprising:
a solid wax whose content in the stick-type cosmetic is more than or equal to 5% by weight and less than or equal to 8% by weight, and
wherein hardness of the stick-type cosmetic is more than or equal to 0.1 and less than or equal to 0.4, and
gloss of the stick-type cosmetic is more than or equal to 30.

2. The stick-type cosmetic according to claim 1,
wherein the solid wax includes polyethylene wax.

3. The stick-type cosmetic according to claim 1,
wherein the stick-type cosmetic is a cosmetic for a lip.

4. A method of manufacturing a stick-type cosmetic, comprising:
heating and melting a composition for the stick-type cosmetic in which the content of a solid wax is more than or equal to 5% by weight and less than or equal to 8% by weight;
supplying the heated and melted composition for stick-type cosmetic into an elastic mold; and
cooling the composition for the stick-type cosmetic in the elastic mold,
wherein a product of the thickness and the elastic coefficient of the elastic mold is more than or equal to 0.15 N/mm and less than or equal to 0.5 N/mm.
